# EUROPEAN PATENT SPECIFICATION

(11) **EP 3 313 513 B1**
(45) Date of publication and mention of the grant of the patent: **10.12.2025**
(21) Application number: 16818371.3
(22) Date of filing: 03.03.2016
(51) Int. Cl.: A61N 5/06, A61B 18/00

(54) **LIGHT-BASED VAGINAL LIGHT THERAPY DEVICE**
LICHTBASIERTE VAGINALE LICHTTHERAPIEVORRICHTUNG
DISPOSITIF DE LUMINOTHÉRAPIE VAGINALE BASÉ SUR LA LUMIÈRE

(30) Priority: 02.07.2015 US 201514789992
(43) Date of publication of application: 02.05.2018
(73) Proprietor: Klang, Gregg, Alan, Coto De Caza, CA 92679 (US)
(72) Inventor: Klang, Gregg, Alan, Coto De Caza, CA 92679 (US)
(74) Representative: Boult Wade Tennant LLP
(86) International application number: PCT/US2016/020532
(87) International publication number: WO 2017/003526

(56) References cited:
- WO-A1-2014/166993
- WO-A2-2006/103678
- TW-A- 200 841 896
- US-A1- 2009 319 008
- US-A1- 2011 190 689
- US-A1- 2011 224 584
- US-A1- 2014 235 942
- US-B1- 6 416 779
- US-B2- 8 109 981

## Description

### BACKGROUND

### Technical Field of Invention

The embodiments herein generally relate to a medical device and particularly relate to light-based vaginal light therapy device for treatment of bacterial and fungal infections.

### Description of Related Art

Vaginitis is characterized by the inflammation of the vagina that results in discharge, itching and pain. The cause is usually a change in the normal balance of vaginal bacteria or an infection. Vaginitis can also result from reduced estrogen levels after menopause. In a given year as many as 50% of the woman female population experiences bacterial or fungal infection within their vagina. The symptoms range from mucus like discharge, itching, aching, pain during intercourse to odor. The vaginal infections often have multiple causes that present challenging cases for treatment. It is critical to have a balance between naturally occurring yeast and bacteria. It is when the system is out of balance or other types of bacteria are present within the environment does one end up with vaginitis. Indeed, when one cause is treated, the other pathogens become resistant or get mutated when treated with anti-biotic and become resistant to anti-biotic therapies. Sometimes the reduction in good bacteria allows for a propagation of yeast, typically *Candida albicans* resulting in yeast infection. Further, either a change in pH balance or introduction of foreign bacteria in the vagina leads to infectious vaginitis. Physical factors that contribute to the development of an infection include the following: constantly wet vulva due to tight clothing, chemicals coming in contact with the vagina via scented tampons, antibiotics, birth control pills, or a diet favoring refined sugar and yeast.

Bacterial vaginosis also known as vaginal bacteriosis or Gardnerella Vaginitis is a disease of the vagina caused by excessive bacteria growth. Common symptoms include increased vaginal discharge that often smells fishlike. The discharge is usually white or gray in color. Burning with urination may also occur. Itching is uncommon. Occasionally there may be no symptoms. Having bacterial vaginosis increases the risk of infection by a number of other sexually transmitted infections including HIV/AIDS. It also increases the risk of early delivery among pregnant women. Bacterial vaginosis is caused by an imbalance of the naturally occurring bacteria in the vagina. Diagnosis is suspected based on the symptom and may be verified by testing the vaginal discharge and finding a higher than normal vaginal pH and large numbers of bacteria. Bacterial vaginosis is often confused with a vaginal yeast infection. Usually treatment is through the use of antibiotics. Bacterial vaginosis is the most common vaginal infection in women of reproductive age. The percentage of women affected at any given time varies between can be as high as 70%. Antibiotics, administered either orally or vaginally are effective in treatment. About 10% to 15% of people, however, do not improve with the first course of antibiotics and recurrence rates of up to 80% have been documented. Recurrence rates are increased with sexual activity with the same pre-post treatment partner and inconsistent condom use although estrogen-containing contraceptives decrease recurrence. There is evidence of an association between Bacterial vaginosis and increased rates of sexually transmitted infections such as HIV/AIDS. Bacterial vaginosis is associated with up to a six-fold increase of HIV shedding. There is also a correlation between the absence of vaginal *lactobacilli* and infection of *Neisseria gonorrhoeae* and *Chlamydia trachomatis.* Bacterial vaginosis is a risk factor for viral shedding and herpes virus type-2 infection. Bacterial vaginosis may increase the risk infection or reactivation of HPV.

Candidiasis, more commonly referred to as a Yeast Infection, is most commonly caused by an overgrowth of a fungus called *Candida albicans* in the vagina. Candida is yeast, a type of fungus. Yeast is always present in the vagina in small numbers, and symptoms only appear with overgrowth. Candida can multiply when an imbalance occurs, such as when the normal acidity of the vagina changes or when hormonal balance changes. Frequently occurring yeast infections may be a sign of more serious overarching health problem such as diabetes or a compromised immune system. Recurrent infections may also be due to use of antibiotic medications. Recurrent vulvovaginal candidiasis affects at least 75 million women annually in the U.S. About 5-8% of women experience four or more episodes per year, diagnosed as recurrent vulvovaginal candidiasis. About 70% of all premenopausal women develop thrush at some point in their lives. With the introduction of over-the-counter medications for home treatment of yeast infections, many women elect to self-diagnose and self-medicate, indicating that the true incidence of yeast infections annually may be significantly under-reported.

In comparison to antibacterial therapy, antifungal treatment is limited to a very small number of drug substances. Treatment for fungal infection can be topical or systemic. Topical antifungals are generally considered as first-line therapy for uncomplicated, superficial, relatively localized fungal infections due to their high efficacy and low potential for systemic adverse effects. Systemic antifungal agents are absorbed and delivered to the body through the blood stream. The oral route is usually the safest, the most economical, and the easiest route for systemic antifungal drugs.

Topical antifungal creams and suppositories have fewer side effects than oral antifungal medications because they aren't absorbed as readily, systemically by the body, and only exert a localized effect on the genital region. Antifungal pills affect the entire body, and side effects can include nausea, headaches, and abdominal pain. However, topical medications can be messy and uncomfortable, while pills are comparatively simple. Treatment using antifungal medication is ineffective in up to 20% of cases. Treatment for thrush is considered to have failed if the symptoms do not clear within 7-14 days. **In** addition, the incidence of resistance to antifungal agents may be increasing, with drug-resistant fungal strains becoming increasingly common causes of infection in high-risk patient groups such as HIV/AIDS patients. Accordingly, alternative antifungal strategies are being actively sought.

Severe forms of infection are hard to treat, and frequently require more aggressive and long-term therapy, as is the case with chronic, recurrent cases. Additionally, incomplete treatments often result in drug resistant infections therefore full course of therapy should be adhered to.

Although, light therapy treatment of various bacterial, fungal or viral infection is generally known, a treatment of such infections is generally achieved through chemical or drug therapies. A use of such therapies affects an internal functioning of the vagina and uterus as the chemicals used in the form of paste or gel or liquid result in unwanted chemical reactions that are harsh or result in various complications.

Oral antifungal medications carry the risk of significant side effects, and many patients are allergic to or intolerant of these drugs. Topical solutions can be messy and inconvenient. There are no existing products for the treatment of yeast infections without also requiring medication. Hence there is a need for a product that allows for the treatment of yeast and bacterial infections quickly and simply without systemic effects. With the continued and accelerating emergence of antibiotic-resistant microorganisms, there is burgeoning interest and investment in light therapy. A device that leverages this rising technology could potentially gain rapid acceptance in specific use cases as well as broader support among the general population simply wishing to avoid exposure to additional medications.

WO 2006/103678 describes a probe device to facilitate reconstitution of tissue lining walls of body cavities that includes a cylindrical probe member having a distal end to be located at the body cavity orifice, a proximal end by which it is inserted into a body cavity, and a handle portion located at the distal end that is connectable to an external control unit 30.

WO 2014/166993 describes a device for photodynamic treatment of the cervix that includes a first housing part for holding a power source and a second housing part that includes an outer portion that forms a hollow frustoconical shape extending away from the first housing part intended to face the user's cervix as well as an LED lamp system and lens, which faces forward in use and for placement against the cervix.

In the view of the foregoing, there is a need for a device to treat the intravaginal infections without creating any harmful side effects. Further, there is a need for a simple device having a miniature size and the ability to maneuver the not-so-easily accessible areas in a vaginal canal.

The above mentioned shortcomings, disadvantages and problems are addressed herein, as detailed below.

### SUMMARY

Various aspects and embodiments of the invention are set out in the appended claims.

The embodiments herein generally relate to a medical device and particularly relate to light-based vaginal light therapy devices for treatment of bacterial and fungal infections.

In accordance with an example, a device is provided for vaginal light therapy of a patient that includes a body sized for introduction into a vagina and including a proximal end and a distal end; one or more light sources carried on the body, each light source configured to emit light outwardly from the body at one or more wavelengths within a range of non-UV germicidal light; and a tether connected with the body and configured for retrieving the device from a vagina of a patient. In an example, the body has an elliptical or oblong shape, e.g., including rounded proximal and distal ends.

Optionally, the device may include an atraumatic cervix support on the distal end of the body configured for placement against a vaginal cervix, e.g., defining a concave recess in the distal end. In addition, the device may include a controller within the body for controlling operation of the one or more light sources and/or a switch, e.g., a pressure-activated switch, for activating and deactivating the one or more light sources.

The one or more light sources may include a plurality of LEDs or other lights mounted to the body, e.g., mounted substantially flush with an outer surface of the body. In another embodiment, one or more internal light sources may be provided within the body and a plurality of lenses may be mounted substantially flush with the outer surface of the body.

The controller may operate the one or more light sources substantially continuously when activated, may automatically turn the one or more light sources off after a predetermined treatment period, and/or may pulse the one or more light sources, e.g., repeatedly activate and deactivate the one or more light sources and/or alternate the one or more light sources between different wavelengths to enhance treatment.

In accordance with another embodiment, a system is provided for vaginal light therapy of a patient that includes a body sized for introduction into a vagina and including a proximal end and a distal end, one or more light sources carried on the body, each light source configured to emit light outwardly from the body at one or more wavelengths within a range of non-UV germicidal light, and a tether connected with the body and configured for retrieving the device from a vagina of a patient; and a photosensitizer configured to be activated by the one or more light sources.

In accordance with still another embodiment, a system is provided for vaginal light therapy of a patient that includes a body sized for introduction into a vagina and including a proximal end and a distal end, one or more light sources carried on the body, each light source configured to emit light outwardly from the body at one or more wavelengths within a range of non-UV germicidal light, and a tether connected with the body and configured for retrieving the device from a vagina of a patient; and a testing strip for determining a type of infection suffered by a patient.

In accordance with yet another embodiment, a method is provided for vaginal light therapy of a patient that includes inserting a body into a vagina such a tether extending from the body exits the vagina; and activating one or more light sources carried on the body, each light source emitting light outwardly from the body at one or more wavelengths within a range of non-UV germicidal light. After treatment, the body may be removed from the vagina, e.g., using the tether.

In another exemplary embodiment, an LED-based vaginal light therapy device is provided for a variety of bacterial and fungal infections. The device may include an LED body, a single or a plurality of LEDs, a switch, a tether, a microchip or other controller, and a battery. The LED body is made up of an appropriate medical grade material, which allows the therapeutic light to be emitted. One end of the device may include a cervix support to place the device smoothly against the cervix. A plurality of LEDs is provided over the LED body. The LED body comprises at-least one LED and each LED emits a light with a wavelength in a therapeutic zone of light in a range of blue and/or red light wavelength. The light emitted is not in the range UV wavelength. The switch is a pressure activated switch. The microchip is housed within the LED body and connects a battery to the single or plurality of LEDs and is further connected to the switch. The microchip controls the duration of light therapy and is also used to pulse the light. A pulsing mechanism of light may be used to treat the targeted bacteria or yeast, e.g. to stress the bacteria or yeast, which may enhance the effectiveness of treatment using the device. The switch and the LEDs draw power from the battery through the microchip. The switch controls an activation as well as deactivation of the plurality of LEDs. The tether is connected to one end of the LED body and is used for retreating and/or progressing the device.

According to one embodiment herein, the plurality of LEDs emit non-UV germicidal light with a wavelength ranging between a blue light wavelength and/or a red light wavelength or a Violet light wavelength. For example, the LEDs may emit light in the range of 405 nm - 470 nm, according to one embodiment herein. The LEDs emit light in the range of 620 nm - 750 nm, according to another embodiment herein. The LEDs emit light in the range of 380 nm - 450 nm, according to another embodiment herein. The emitted light may kill or limit propagation of various strains of bacteria and fungus.

According to another embodiment herein, the microchip controls the duration of light pulse in a rapid on and/or off manner.

According to one embodiment herein, the hardened material forming the LED body is a medical grade plastic.

According to one embodiment herein, the LED body is sealed to avoid a flow of vaginal fluid into the device.

According to one embodiment herein, the device is non-reusable in nature and serves a treatment of bacterial and fungal infection for single use.

According to one embodiment herein, the device is reusable in nature and serves a treatment of bacterial and fungal infection for multiple times. The reusable device has a washable or rinse-able LED body. The re-usable device incorporates a mini-USB cable appropriate for use as a tether and for recharging the device.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation. Many changes and modifications may be made within the scope of the embodiments herein without departing from the spirit thereof, and the embodiments herein include all such modifications.

### BRIEF DESCRIPTION OF THE DRAWINGS

The other objects, features and advantages will occur to those skilled in the art from the following description of the preferred embodiment and the accompanying drawings in which:
FIG. 1A illustrates a perspective view of a light-based vaginal light therapy device, according to one embodiment herein.
FIG. 1B illustrates a perspective view of a light-based vaginal light therapy device with a USB cord, according to one embodiment herein.
FIGS. 1C, 1D, and 1E are top, side, and front views, respectively, of the device of FIG. 1B.
FIG. 1F illustrates a sectional view of the light-based vaginal light therapy device of FIG. 1B.
FIG. 2 illustrates a placement of a vaginal light therapy device inside a vaginal canal of a female, according to one embodiment herein.

### DETAILED DESCRIPTION OF THE EXEMPLARY EMBODIMENTS

In the following detailed description, a reference is made to the accompanying drawings that form a part hereof, and in which the specific embodiments that may be practiced is shown by way of illustration. The embodiments are described in sufficient detail to enable those skilled in the art to practice the embodiments and it is to be understood that the logical, mechanical and other changes may be made without departing from the scope of the embodiments. The following detailed description is therefore not to be taken in a limiting sense.

The embodiments herein provide a light-based vaginal light therapy device for treating a variety of bacterial and fungal infections. In an exemplary embodiment, the device may include a body or housing, a single or a plurality of light sources, a switch, a tether, microchip or other controller, and a battery or other power source. The body is made up of an appropriate medical grade material which allows the therapeutic light to be emitted. Optionally, one end of the device may include a cervix support to place the device smoothly against the cervix. In an exemplary embodiment, the light source(s) may include a single or multiple LEDs provided over the LED body, e.g., mounted to the outer surface, within recesses in the outer surface, and the like, e.g., to provide a substantially smooth and/or atraumatic outer surface for the device. Alternatively, other internal light sources may be provided that may transmit light from the body, e.g., via one or more fiber optics, lenses, and the like (not shown).

Each LED may emit a light with a wavelength in a therapeutic zone of light in a range of blue and/or red light or Violet light (germicidal non UV) wavelength. The light emitted is not in the range UV wavelength. In an exemplary embodiment, the switch is a pressure activated switch. The controller is housed within the body and connects a battery to the light source(s) and is further connected to the switch. The controller may control the duration of light therapy and/or pulse the light. For example, pulsing the light may stress the targeted bacteria or yeast and/otherwise make the device more effective. The switch and the light source(s) draw power from the battery through the microchip. The switch controls an activation as well as deactivation of the light source(s).

The tether may be connected to one end of the body and may have sufficient length to facilitate retrieving and/or progressing the device from a vagina. Alternatively, a cannula or other insertion tool (not shown) may be provided for inserting and/or retrieving the device.

According to one embodiment herein, the light source(s) may emit a non-UV germicidal light with a wavelength ranging between a blue light wavelength and/or a red light wavelength or a Violet light wavelength. For example, LEDs may be used that emit light in the range of 405 nm - 470 nm, according to one embodiment herein. Alternatively, the LEDs emit light in the range of 620 nm - 750 nm, or the LEDs may emit light in the range of 380 nm - 450 nm. The emitted light may kill or limit propagation of various strains of bacteria and fungus.

According to one embodiment herein, the controller controls the duration of light pulse in a rapid on and/or off manner. For example, once activated, the controller may maintain the light source(s) active for a predetermined time period, e.g., an hour or more, and then automatically deactivate the light source(s). Optionally, when the device is activated, the controller may pulse the light source(s), e.g., rapidly turning the light source(s) off and on multiple times per minute or per second. In addition or alternatively, the light source(s) may be pulsed between different wavelengths.

According to one embodiment herein, the hardened material forming the body is a medical grade plastic.

According to one embodiment herein, the body is sealed to avoid a flow of vaginal fluid into the device.

According to one embodiment herein, the device is non-reusable in nature and serves a treatment of bacterial and fungal infection for single use. Further, the device is reusable in nature and serves a treatment of bacterial and fungal infection for multiple times by using a mini-USB cable as a tether and for recharging the device, according to another embodiment herein. The reusable device has a washable or rinse-able body.

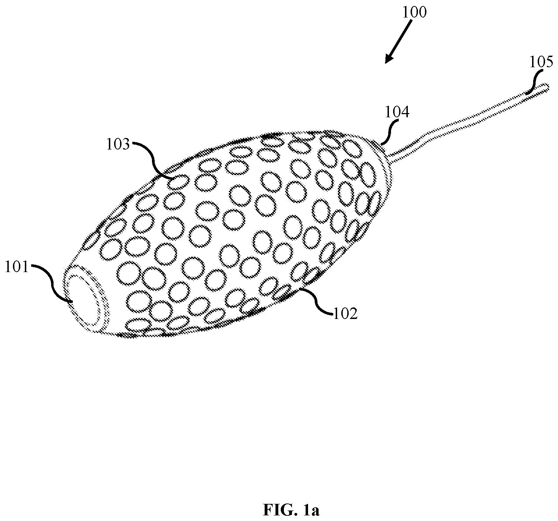

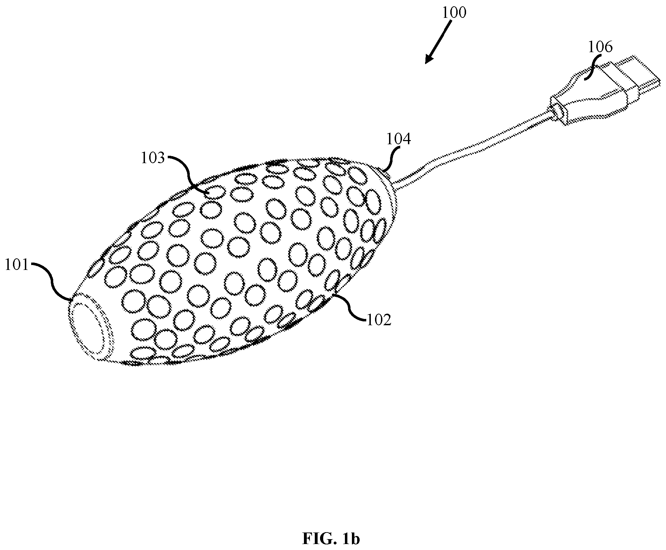

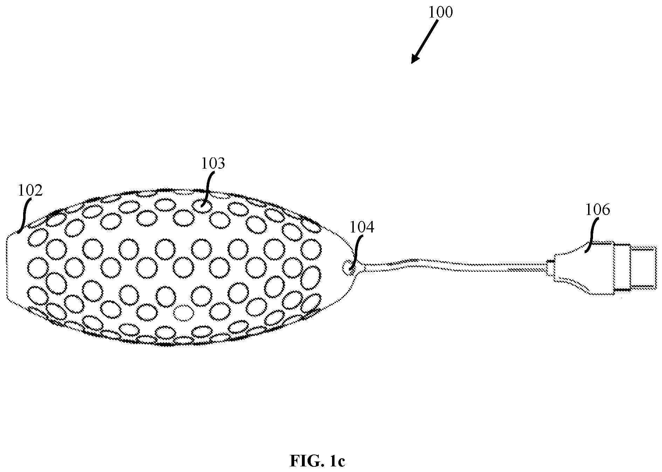

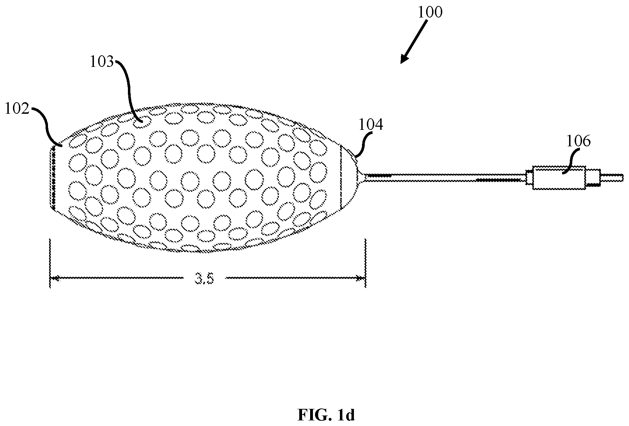

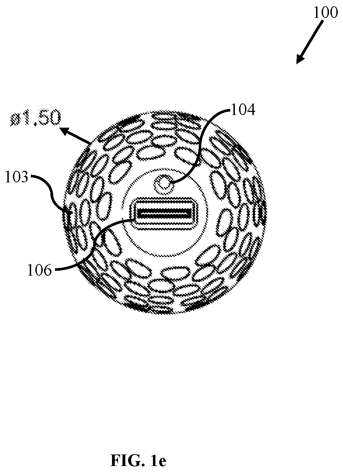

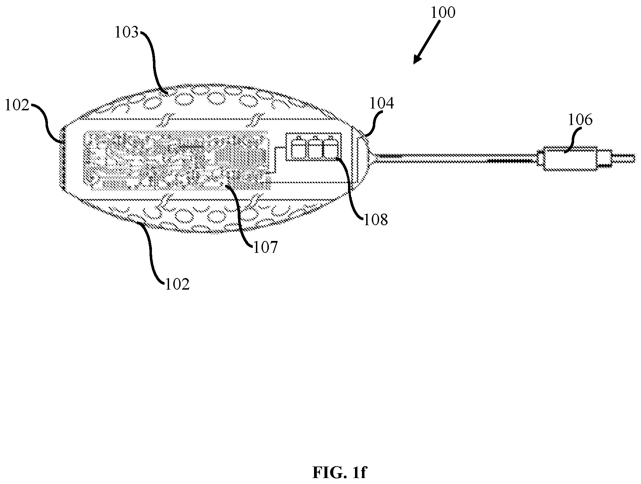

Turning to the drawings, FIG. 1A illustrates a perspective view of an exemplary embodiment of a LED-based vaginal light therapy device 100 including a body 102 carrying a plurality of light sources 103. FIG. 1B illustrates a perspective view of the LED-based vaginal light therapy device 100 with a USB cord 106, according to one embodiment herein. FIGS. 1C-1F illustrates a top view, a side view, a front view and a sectional view respectively of the LED-based vaginal light therapy device 100, according to one embodiment herein. With respect to FIGS. 1A-1F, the body 102 of the light therapy device 100 is primarily a plastic framework which allows positioning of single or multiple LEDs 103 on an external surface. As shown in FIG. 1F, a battery 108, controller 107, and/or additional electronic controllers and circuits are positioned internally with respect to the LEDs 103.

A switch 104 may be located on the outer surface of the body 102, which may be actuated by the user to activate the device 100, e.g., before insertion into the vagina. Alternatively, the tether 105 may be coupled to a switch within the body such that the tether 105 may be pulled or otherwise manipulated to activate/deactivate the light sources 104. In a further alternative, a pressure-activated switch (not shown) may be provided within the body 102 that is responsive to compressive pressures on the body 102, e.g., such that the device 100 may automatically activated once inserted into the vagina and the pressure from the surrounding muscles compress the body 102.

The device 100, once assembled, is encased into an appropriate medical grade plastic housing which is completely sealed until not serviceable. A suitable tether 105, as shown in FIG. 1A, or a mini-USB cable 106, as shown in FIG. 1B, may be attached at a first or proximal end of the body 102 to assist in insertion and/or retrieval of the device 100 inwards or outwards of the vaginal canal. Optionally, a second or distal end of the device 100 may include a cervix support 101, e.g., a concave or otherwise shaped recess to place the device smoothly against the cervix.

**In** an exemplary embodiment, the length and diameter of the body may be sized for insertion fully into a vagina, e.g., having an elliptical or oblong shape, i.e., with the length greater than the diameter. For example, the body 100 may have a length not more than about 3.5 inches and a diameter of not more than about 1.5 inches. The surface of the body 102 is either rigid or squeezable depending on the basis of user preference and area of usage.

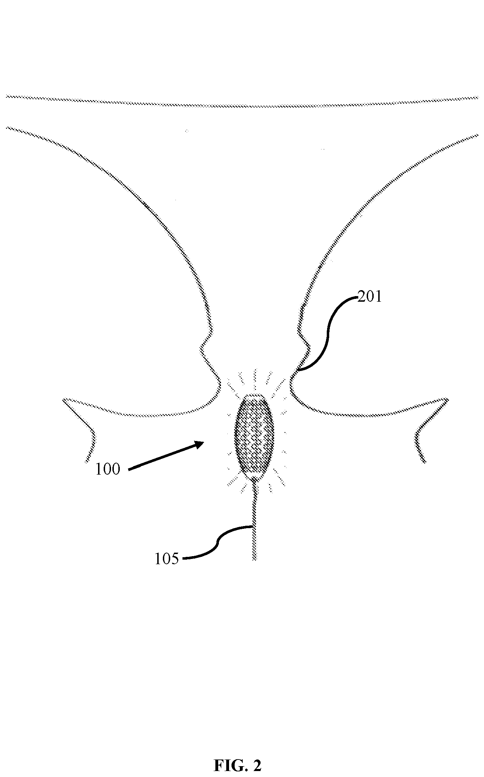

FIG. 2 illustrates a placement of the vaginal light therapy device inside the vaginal canal of a female, according to one embodiment herein. With respect to FIG. 2, the device 100 is inserted into the vaginal canal 201with the tether 105 or USB cable 106 extending from the canal. The device 100 is activated by actuating the switch 104 before inserting the device 100 into the vagina, or the device 100 may be pressure-activated, e.g., after it reaches a predetermined position in the vaginal canal 201, whereupon the light source(s) start emitting the light. The device 100 is left in the vagina for a specific period of time varying from a few minutes to hours depending upon extent of infection and kind of infection (bacterial or fungal).

The light therapy device 100 disclosed herein may provide a harmless and/or efficient treatment of the intravaginal infection. Since the device 100 does not react with any vaginal fluid, the device 100 may be used in any patient's condition. Also the device 100 may have a relatively low cost and easy usage procedure, so it is usable even personally after a physician's approval.

According to an embodiment herein, the device may be useful for the treatment of fungal and bacterial vagintis. **In** case of bacterial vaginitis, there is no need for the use of additional photo sensitizing agents as bacteria are negatively affected by the light based therapy of the device 100.

The device 100 may help to eliminate or reduce undesirable microorganisms as an adjunct and forms a basis for the replacement of traditional therapies. The device 100 may also be useful for patients who are interested in non-drug therapies. The patients who cannot tolerate oral or topical azole therapy, as well as immune-compromised patients with recurrent yeast or bacterial infections can be treated with the device 100.

According to one embodiment herein, the device may be useful against fungal as well as bacterial infections. The fungal infection comprises the infection caused by yeast and especially by *Candida albicans* while the bacterial infection comprises the infection caused principally by *Gardnerella.* The patient has to determine first whether he is suffering from a fungal infection or a bacterial infection. This can be determined first through a doctor's test.

According to an embodiment herein, the device is sold along with a testing strip, e.g., as part of a kit or system for treatment. The testing strip may be used for the determination of the fungal and the bacterial infection suffered by a patient according to the embodiments herein. The bacterial as well as fungal infections may be treated using the device 100 as an alternative to drugs, douches or chemicals prescribed by a doctor.

According to another embodiment herein, in case of fungal infection, the device may be used along with a photo-sensitizer. The photo-sensitizer may be beneficial in cases of yeast infection. The photo sensitizer comprises porfimer sodium (Photofrin), 5-aminolevulinic acid or ALA (Levulan), and methyl aminolevulinate [MAOP] (Metvix).

According to one embodiment herein, the device 100 may provide a low power long duration therapy so as to be safer for the mucosal tissue. The idea is that the device can be inserted overnight and pulled in the morning. The LEDs are single color or multi-color LEDs, pulsed or non-pulsed lights.

According to one embodiment herein, the device 100 is made as a single use device.

According to another embodiment herein, the device 100 is made for multiple usages, e.g., such that the device 100 may be cleaned and inserted into the vagina multiple times, e.g., over several days or other course of treatment. For multiple usages, the device 100 may include a rechargeable battery and a cord which facilitates the removal of the device from the vagina as well as acts as a connection with a suitable power source in order to recharge the device.

According to one embodiment herein, the light therapy device comprises one or more LEDs as light source for impending light on the vaginal walls. The device further comprises a battery housed inside the 100% sealed housing or the LED body. The battery may be connected to and act as a power source to the controller as well as the LEDs. The microchip controls a duration of the light therapy. A printed circuit or a suitable electronic circuitry or hub may be provided in the device for interconnecting the switch, the LEDs, the microchip and the battery. The device further comprises switch activates a device to start the light therapy. The device also comprises a tether for retrieval of the device during a light therapy. The tether is suitably replaced by a USB cord or a charging cord for making device suitable for multiple usage.

According to the embodiments herein, the device may be useful to kill or render inert the targeted species which keeps the species from replicating. The device may also be used as an adjunct therapy with existing known treatments possibly allowing for a reduction in drug or chemical based therapies. If the device is used with the conventional therapies then the device is likely to reduce the treatment times.

Thus, the device may provide a non-drug based alternative therapy based on safe and germicidal light which when introduced into the region provides a safe and effective method to treat and control both Yeast and Bacterial infection. In some applications, the device effectiveness may be enhanced through the use of a photo-sensitizer. For example, a photo-sensitizer may be applied to the outer surface of the body 102 or into pockets or features (not shown) configured for receiving the phot-sensitizer. Alternatively, the photo-sensitizer may be introduced separately into the vagina, e.g., using known applicators (not shown). The device may be used in conjunction with standard systemic drug or topical cream based therapies to lessen the duration of the event.

It is to be understood that the phraseology or terminology employed herein is for the purpose of description and not of limitation. Therefore, while the embodiments herein have been described in terms of exemplary embodiments, those skilled in the art will recognize that the embodiments herein can be practiced with modification within the scope of the claims.

## Claims

1. A device (100) for vaginal light therapy of a patient for treatment of bacterial and fungal infections, comprising:
a body (102) sized for introduction into a vagina and including a proximal end and a distal end;
one or more light sources (103) carried on or within the body, each light source configured to emit light outwardly from the body at one or more wavelengths within a range of non-UV germicidal light;
a controller (107) positioned within the body for controlling operation of the one or more light sources;
a rechargeable battery (108) positioned within the body; and
a cord (105) configured to facilitate the removal of the device from a vagina of a patient as well as being configured to act as a connection with a suitable power source in order to recharge the battery;
wherein the body has an elliptical shape including rounded proximal and distal ends, and
wherein the body has a length and diameter sized for insertion fully into a vagina.

2. The device of claim 1, wherein the one or more light sources comprise one or more internal light sources within the body and a plurality of lenses mounted flush with an outer surface of the body.

3. The device of claim 1, further comprising an atraumatic cervix support (101) on the distal end of the body configured for placement against a vaginal cervix.

4. The device of claim 1, wherein the body further comprises pockets configured for receiving a photo-sensitizer.

5. The device of claim 1, wherein the controller is configured to activate the one or more light sources for a predetermined treatment period whereupon the controller automatically deactivates the one or more light sources.

6. The device of claim 1, wherein the controller is configured to activate the one or more light sources to pulse at a predetermined rate.

7. The device of claim 1, wherein the controller is configured to activate the one or more light sources to emit different wavelengths in a predetermined sequence.

8. The device of claim 1, further comprising a switch (104) coupled to the one or more light sources for selectively activating the one or more light sources.

9. The device of claim 8, wherein the switch is configured to control activation of the one or more light sources based at least in part on pressure sensing intravaginal walls of a vagina within which the device is introduced.

10. The device of claim 1, wherein the cord is a mini-USB cable or a USB cable .

11. The device of claim 1,
wherein the body is made up of a hardened material;
further comprising a cervix support, wherein the cervix support is provided at an end of the body to place the device smoothly against a vaginal cervix;
wherein the one or more light sources comprise one or more LEDs present on the body, wherein each LED emits a light having a wavelength in a range of non-UV germicidal light;
a switch, wherein the switch is a pressure activated switch present on the LED body;
wherein the controller comprises a microchip housed within the body, wherein the microchip connects the battery (108) to the one or more LEDs, wherein the microchip is further connected to the switch;
wherein the cord is connected with the body and is used for retreating and/or progressing the device through a vagina of a patient;
wherein, the switch and the one or more LEDs draw a power from the battery through the microchip, wherein the switch controls an activation as well as deactivation of the one or more LEDs on the basis of a pressure sensing by the intravaginal walls.

12. The device according to any preceding claim, wherein the non-UV germicidal light comprises a range of blue light with 405 nm - 470 nm wavelength, a range of red light with 620 nm - 750 nm wavelength, or a range of Violet light with 380-450 nm wavelength.

13. The device according to claim 11, wherein the microchip controls a duration of light pulse in a rapid on and/or off manner.

14. A system for vaginal light therapy of a patient, comprising:
a device according to any preceding claim; and
a photosensitizer configured to be activated by the one or more light sources.

15. A system for vaginal light therapy of a patient, comprising:
a device according to any of preceding claims 1-13 ; and
a testing strip for determining a type of infection suffered by a patient.

## Patentansprüche

1. Vorrichtung (100) zur vaginalen Lichttherapie einer Patientin zur Behandlung von bakteriellen und Pilz-Infektionen, umfassend:
einen Körper (102), welcher zum Einführen in eine Vagina dimensioniert ist und ein proximales Ende und ein distales Ende umfasst;
eine oder mehrere Lichtquellen (103), welche an oder innerhalb des Körpers getragen sind, wobei jede Lichtquelle dazu eingerichtet ist, Licht nach außen aus dem Körper zu emittieren bei einer oder mehreren Wellenlängen innerhalb eines Bereichs von nicht-UV germizidem Licht;
eine Steuereinheit (107), welche innerhalb des Körpers positioniert ist, um den Betrieb der einen oder mehrerer Lichtquellen zu steuern;
eine wiederaufladbare Batterie (108), welche innerhalb des Körpers positioniert ist; und
ein Kabel (105), welches dazu eingerichtet ist, das Entfernen der Vorrichtung aus einer Vagina einer Patientin zu erleichtern, sowie dazu eingerichtet ist, als Verbindung mit einer geeigneten Stromquelle zum Aufladen der Batterie zu dienen;
wobei der Körper eine elliptische Form mit abgerundeten proximalen und distalen Enden aufweist, und
wobei der Körper eine Länge und einen Durchmesser, für ein vollständiges Einführen in eine Vagina dimensioniert, aufweist.

2. Vorrichtung nach Anspruch 1,
wobei die eine oder mehreren Lichtquellen eine oder mehrere interne Lichtquellen innerhalb des Körpers und eine Mehrzahl von Linsen umfassen, welche bündig mit einer äußeren Fläche des Körpers montiert sind.

3. Vorrichtung nach Anspruch 1,
ferner umfassend eine atraumatische Zervixstütze (101) an dem distalen Ende des Körpers, welche zum Platzieren gegen eine vaginale Zervix eingerichtet ist.

4. Vorrichtung nach Anspruch 1,
wobei der Körper ferner Taschen umfasst, welche dazu eingerichtet sind, einen Photosensibilisator aufzunehmen.

5. Vorrichtung nach Anspruch 1,
wobei die Steuereinheit dazu eingerichtet ist, die eine oder mehreren Lichtquellen für eine vorbestimmte Behandlungsperiode zu aktivieren, woraufhin die Steuereinheit automatisch die eine oder mehreren Lichtquellen deaktiviert.

6. Vorrichtung nach Anspruch 1,
wobei die Steuereinheit dazu eingerichtet ist, die eine oder mehreren Lichtquellen zu aktivieren, um mit einer vorbestimmten Rate zu pulsieren.

7. Vorrichtung nach Anspruch 1,
wobei die Steuereinheit dazu eingerichtet ist, die eine oder mehreren Lichtquellen zu aktivieren, um verschiedene Wellenlängen in einer vorbestimmten Sequenz zu emittieren.

8. Vorrichtung nach Anspruch 1,
ferner umfassend einen Schalter (104), welcher mit der einen oder den mehreren Lichtquellen gekoppelt ist, um die eine oder mehreren Lichtquellen selektiv zu aktivieren.

9. Vorrichtung nach Anspruch 8,
wobei der Schalter dazu eingerichtet ist, ein Aktivieren der einen oder mehreren Lichtquellen wenigstens teilweise basierend auf einem Druckerfassen von intravaginalen Wänden einer Vagina zu kontrollieren, in welche die Vorrichtung eingeführt ist.

10. Vorrichtung nach Anspruch 1,
wobei das Kabel ein Mini-USB-Kabel oder ein USB-Kabel ist.

11. Vorrichtung nach Anspruch 1,
wobei der Körper aus einem gehärteten Material besteht;
ferner umfassend eine Zervixstütze, wobei die Zervixstütze an einem Ende des Körpers bereitgestellt ist, um die Vorrichtung leichtgängig gegen eine vaginale Zervix zu platzieren;
wobei die eine oder mehreren Lichtquellen eine oder mehrere LEDs umfassen, welche an dem Körper vorhanden sind, wobei jede LED Licht mit einer Wellenlänge in einem Bereich von nicht-ultraviolettem germizidem Licht emittiert;
einen Schalter, wobei der Schalter ein druckaktivierter Schalter ist, welcher an dem LED-Körper vorhanden ist,
wobei die Steuereinheit einen Mikrochip umfasst, welcher in dem Körper aufgenommen ist, wobei der Mikrochip die Batterie (108) mit der einen oder den mehreren LEDs verbindet, wobei der Mikrochip ferner mit dem Schalter verbunden ist;
wobei das Kabel mit dem Körper verbunden ist und zum Zurückziehen und/oder Fortschreiten der Vorrichtung durch eine Vagina einer Patientin verwendet wird;
wobei der Schalter und die eine oder mehreren LEDs Energie aus der Batterie über den Mikrochip beziehen, wobei der Schalter eine Aktivierung sowie Deaktivierung der einen oder mehrerer LEDs basierend auf einem Druckerfassen durch die intravaginalen Wände steuert.

12. Vorrichtung nach einem vorhergehenden Anspruch,
wobei das nicht-UV germizide Licht einen Bereich von blauem Licht mit einer Wellenlänge von 405 nm - 470 nm, einen Bereich von rotem Licht mit einer Wellenlänge von 620 nm - 750 nm oder einen Bereich von violettem Licht mit einer Wellenlänge von 380 - 450 nm umfasst.

13. Vorrichtung nach Anspruch 11,
wobei der Mikrochip eine Dauer eines Lichtpulses in einer schnellen An- und/oder Aus-Weise steuert.

14. System zur vaginalen Lichttherapie einer Patientin, umfassend:
eine Vorrichtung nach einem vorhergehenden Anspruch; und
einen Photosensibilisator, welcher dazu eingerichtet ist, durch die eine oder mehreren Lichtquellen aktiviert zu werden.

15. System zur vaginalen Lichttherapie einer Patientin, umfassend:
eine Vorrichtung nach einem der vorhergehenden Ansprüche 1 bis 13; und
einen Teststreifen zum Bestimmen eine Art von Infektion, an welcher einer Patientin leidet.

## Revendications

1. Dispositif (100) de luminothérapie vaginale d'une patiente pour le traitement d'infections bactériennes et fongiques, comprenant :
un corps (102) dimensionné pour une introduction dans un vagin et comprenant une extrémité proximale et une extrémité distale ;
une ou plusieurs sources de lumière (103) portées sur ou à l'intérieur du corps, chaque source de lumière étant configurée pour émettre de la lumière vers l'extérieur à partir du corps à une ou plusieurs longueurs d'onde dans une plage de lumière germicide non UV ;
un dispositif de commande (107) positionné à l'intérieur du corps pour commander le fonctionnement des une ou plusieurs sources de lumière ;
une batterie rechargeable (108) positionnée à l'intérieur du corps ; et
un cordon (105) configuré pour faciliter le retrait du dispositif à partir d'un vagin d'une patiente ainsi que configuré pour agir comme une connexion avec une source d'alimentation appropriée afin de recharger la batterie ;
dans lequel le corps présente une forme elliptique comprenant des extrémités proximale et distale arrondies, et
dans lequel le corps présente une longueur et un diamètre dimensionnés pour une insertion complète dans un vagin.

2. Dispositif selon la revendication 1, dans lequel les une ou plusieurs sources de lumière comprennent une ou plusieurs sources de lumière internes à l'intérieur du corps et une pluralité de lentilles montées au ras d'une surface extérieure du corps.

3. Dispositif selon la revendication 1, comprenant en outre un support de col de l'utérus atraumatique (101) sur l'extrémité distale du corps configuré pour être placé contre un col de l'utérus vaginal.

4. Dispositif selon la revendication 1, dans lequel le corps comprend en outre des poches configurées pour recevoir un photosensibilisateur.

5. Dispositif selon la revendication 1, dans lequel le dispositif de commande est configuré pour activer les une ou plusieurs sources de lumière pendant une période de traitement prédéterminée, après quoi le dispositif de commande désactive automatiquement les une ou plusieurs sources de lumière.

6. Dispositif selon la revendication 1, dans lequel le dispositif de commande est configuré pour activer les une ou plusieurs sources de lumière pour qu'elles émettent des impulsions à une vitesse prédéterminée.

7. Dispositif selon la revendication 1, dans lequel le dispositif de commande est configuré pour activer les une ou plusieurs sources de lumière pour émettre différentes longueurs d'onde dans une séquence prédéterminée.

8. Dispositif selon la revendication 1, comprenant en outre un commutateur (104) couplé aux une ou plusieurs sources de lumière pour activer sélectivement les une ou plusieurs sources de lumière.

9. Dispositif selon la revendication 8, dans lequel le commutateur est configuré pour commander l'activation des une ou plusieurs sources de lumière sur la base au moins en partie de parois intravaginales de détection de pression d'un vagin à l'intérieur duquel le dispositif est introduit.

10. Dispositif selon la revendication 1, dans lequel le cordon est un câble mini-USB ou un câble USB.

11. Dispositif selon la revendication 1 ;
dans lequel le corps est composé d'un matériau durci ; comprenant en outre un support de col de l'utérus, dans lequel le support de col de l'utérus est prévu à une extrémité du corps pour placer le dispositif en douceur contre un col de l'utérus vaginal ;
dans lequel les une ou plusieurs sources de lumière comprennent une ou plusieurs diodes électroluminescentes, DEL, présentes sur le corps, dans lequel chaque DEL émet une lumière présentant une longueur d'onde dans une plage de lumière germicide non UV ;
un commutateur, dans lequel le commutateur est un commutateur activé par pression présent sur le corps de DEL ;
dans lequel le dispositif de commande comprend une micropuce logée à l'intérieur du corps, dans lequel la micropuce connecte la batterie (108) aux une ou plusieurs DEL, dans lequel la micropuce est en outre connectée au commutateur ;
dans lequel le cordon est connecté au corps et est utilisé pour retirer et/ou faire progresser le dispositif à travers le vagin d'une patiente ;
dans lequel, le commutateur et les une ou plusieurs DEL tirent une énergie à partir de la batterie à travers la micropuce, dans lequel le commutateur commande une activation ainsi qu'une désactivation des une ou plusieurs DEL sur la base d'une détection de pression par les parois intravaginales.

12. Dispositif selon l'une quelconque des revendications précédentes, dans lequel la lumière germicide non UV comprend une plage de lumière bleue avec une longueur d'onde de 405 nm à 470 nm, une plage de lumière rouge avec une longueur d'onde de 620 nm à 750 nm, ou une plage de lumière violette avec une longueur d'onde de 380 à 450 nm.

13. Dispositif selon la revendication 11, dans lequel la micropuce commande une durée d'impulsion de lumière d'une manière marche et/ou arrêt rapide.

14. Système pour une luminothérapie vaginale d'une patiente, comprenant :
un dispositif selon l'une quelconque des revendications précédentes ; et
un photosensibilisateur configuré pour être activé par les une ou plusieurs sources de lumière.

15. Système pour une luminothérapie vaginale d'une patiente, comprenant :
un dispositif selon l'une quelconque des revendications précédentes 1 à 13, et
une bandelette de test pour déterminer un type d'infection dont souffre une patiente.
